(19) Europäisches Patentamt
European Patent Office
Office européen des brevets

(11) Veröffentlichungsnummer: **0 050 097**
**B1**

(12) # EUROPÄISCHE PATENTSCHRIFT

(45) Veröffentlichungstag der Patentschrift: **05.12.84**

(51) Int. Cl.³: **C 07 D 213/64**, A 01 N 43/40

(21) Anmeldenummer: **81810405.1**

(22) Anmeldetag: **09.10.81**

(54) **Neue alpha-(Pyridyl-2-oxy-phenoxy)-propionsäurederivate, Verfahren zu deren Herstellung, sowie deren Verwendung als Herbizide und/oder Pflanzenwachstumsregulatoren.**

(30) Priorität: **15.10.80 CH 7699/80**
**04.09.81 CH 5720/81**

(43) Veröffentlichungstag der Anmeldung: **21.04.82 Patentblatt 82/16**

(45) Bekanntmachung des Hinweises auf die Patenterteilung: **05.12.84 Patentblatt 84/49**

(84) Benannte Vertragsstaaten: **AT BE CH DE FR GB IT LI NL**

(56) Entgegenhaltungen:
**EP - A - 0 001 473**
**EP - A - 0 002 246**
**EP - A - 0 003 114**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Postfach, CH-4002 Basel (CH)**

(72) Erfinder: **Rempfler, Hermann, Dr., Brücklismattstrasse 16, CH-4107 Ettingen (CH)**
Erfinder: **Böhner, Beat, Dr., Hügelweg 3, CH-4102 Binningen (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

EP 0 050 097 B1

## Beschreibung

Die Erfindung betrifft Verbindungen der Formel I

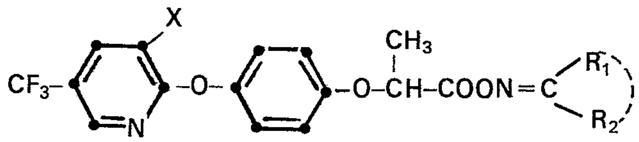 (I),

worin

X für Wasserstoff, Halogen oder Pseudohalogen steht;

$R_1$ Wasserstoff oder eine unsubstituierte oder durch Halogen, Hydroxy oder $C_1$–$C_3$-Alkoxy substituierte $C_1$–$C_6$-Alkyl- oder Phenylgruppe bedeutet;

$R_2$ eine unsubstituierte oder eine durch Halogen, Hydroxy oder $C_1$–$C_3$-Alkoxy substituierte $C_1$–$C_6$-Alkyl, $C_2$–$C_6$-Alkyl, $C_2$–$C_6$-Alkinyl- oder Phenylgruppe bedeutet; oder

$R_1$ und $R_2$ zusammen für eine unsubstituierte oder eine durch $C_1$–$C_3$-Alkyl substituierte Tetramethylen-, Pentamethylen- oder Hexamethylenbrücke stehen,

Verfahren zu deren Herstellung, sie als Wirkstoff enthaltende Mittel sowie deren Verwendung als Herbizide und/oder als Pflanzenwachstumsregulatoren.

Unter Alkyl oder Alkylanteil eines anderen Substituenten sind je nach Zahl der angegebenen Kohlenstoffatome folgende Gruppen zu verstehen: Methyl, Ethyl, Propyl, Butyl, Pentyl, Hexyl, sowie ihre Isomeren, z.B. iso-Propyl, iso-Butyl, sek.-Butyl usw.; Alkenyl steht beispielsweise für Propenyl-(1), Allyl, Butenyl-(1), Butenyl-(2), Butenyl-(3) usw.; unter Alkinyl wird insbesondere Propargyl verstanden.

Halogen steht hier und im folgenden für Fluor, Chlor, Brom oder Jod, bevorzugt für Chlor oder Brom, insbesondere für Chlor. Unter Pseudohalogengruppen sind die Cyano- und Rhodanogruppe, insbesondere die Cyanogruppe zu verstehen.

Die Verbindungen der Formel I sind bei Raumtemperatur stabile Öle, Harze oder Feststoffe, die sich durch sehr wertvolle herbizide und pflanzenwachstumsregulierende Eigenschaften auszeichnen. Sie lassen sich daher bevorzugt auf dem Agrarsektor oder verwandten Gebieten zur gezielten Beeinflussung des Pflanzenwachstums und/oder zur Bekämpfung von Unkräutern einsetzen.

Bevorzugte Gruppen von Wirkstoffen der Formel I bilden diejenigen Verbindungen, bei denen der Substituent X für Chlor, Brom oder auch Wasserstoff steht, und $R_1$ sowie $R_2$ die unter Formel I angegebenen Bedeutungen haben.

Weiterhin werden diejenigen Wirkstoffe der Formel I bevorzugt, bei denen $R_1$ für Wasserstoff oder $C_1$–$C_6$-Alkyl und $R_2$ für $C_1$–$C_6$-Alkyl stehen, während X die unter Formel I angegebenen Bedeutungen hat.

Besonders bevorzugt im Umfang der Formel I sind Wirkstoffe, worin X für Chlor steht, $R_1$ und $R_2$ unabhängig voneinander $C_1$–$C_3$-Alkyl bedeuten.

Folgende Einzelverbindungen werden besonders bevorzugt:

α-[-4-(3-Chlor-5-trifluormethyl-pyridyl-2-oxy)-phenoxy]-propionsäureacetonoximester

α-[4-(3-Chlor-5-trifluormethyl-pyridyl-2-oxy)-phenoxy]-propionsäuremethylethylketonoximester

α-[4-(3-Chlor-5-trifluormethyl-pyridyl-2-oxy)-phenoxy]-propionsäurediethylketonoximester.

Die neuen α-(Pyridyl-2-oxy-phenoxy)-propionsäurederivate der Formel I können nach folgenden, an sich bekannten Methoden hergestellt werden, wobei in den Formeln II bis V die Substituenten X, $R_1$ und $R_2$ die unter Formel I angegebenen Bedeutungen haben, Hal und Hal' für ein Halogenatom, vorzugsweise für Chlor oder Brom stehen und M Wasserstoff oder bevorzugt ein Alkali- oder Erdalkalimetallkation, insbesondere ein Kalium- oder Natriumkation bedeutet.

Ein Weg zur Herstellung der Wirkstoffe der Formel I besteht in der Reaktion eines substituierten Säurehalogenids der Formel II

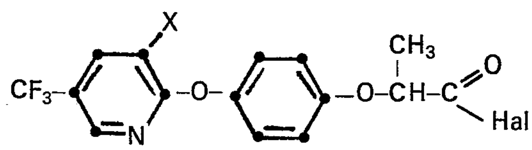 (II)

mit einem Oxim der Formel III

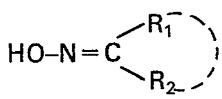 (III)

Weiter kommt man zu den Verbindungen der Formel I durch Reaktion einer Verbindung der Formel IV

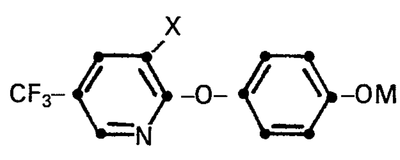 (IV)

mit einem α-Halogenpropionsäureoximester der Formel V

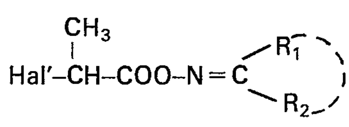 (V)

Schliesslich erhält man Verbindungen der Formel I auch durch Reaktion eines 2-Halogen-5-trifluormethyl-pyridins der Formel VI

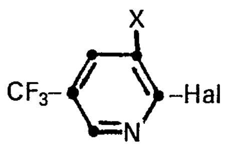

(VI)

mit dem Metallsalz eines Hydrochinon-äthers mit einem Milchsäureoximester der Formel VII

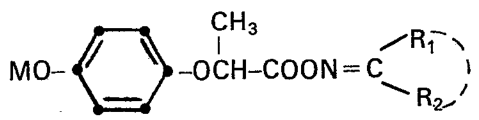

(VII)

In den obigen Formeln haben $R_1$, $R_2$ und X die unter Formel I gegebene Bedeutung, Hal steht für ein Halogenatom, vorzugsweise Chlor oder Brom während M für Wasserstoff oder ein Alkalimetall- oder Erdalkalimetallkation steht.

Diese Reaktionen werden in einem den Reaktionspartnern gegenüber inerten organischen Lösungsmittel vorteilhafterweise in Gegenwart eines säurebindenden Mittels durchgeführt. Als säurebindendes Mittel kommen organische und anorganische Basen in Betracht, z.B. tertiäre Amine wie Trialkylamin (Trimethylamin, Triethylamin, Tripropylamin usw.), Pyridin und Pyridinbasen (4-Dimethylaminopyridin, 4-Pyrrolidylaminopyridin usw.), Oxide und Hydroxide, Alkoholate, Carbonate und Hydrogencarbonate von Alkali- und Erdalkalimetallen (KOH, NaOH, $Na_2CO_3$ usw.) sowie Alkalisalze von Carbonsäuren ($CH_3COONa$ usw.).

Entstehender Halogenwasserstoff kann in manchen Fällen auch mittels Durchleiten von Inertgas, z.B. Stickstoff, aus dem Reaktionsgemisch vertrieben werden oder an Molekularsieb (Porengrösse 3Å bis 5Å) adsorbiert werden [1Å = 0,1 nm].

Alle Umsetzungen werden in Anwesenheit von reaktionsinerten Lösungs- oder Verdünnungsmitteln durchgeführt. In Frage kommen beispielsweise aromatische und aliphatische Kohlenwasserstoffe wie Benzol, Toluol, Xylole, Petrolether, Cyclohexan, n-Hexan; halogenierte Kohlenwasserstoffe wie Chlorbenzol, Methylenchlorid, Ethylenchlorid, Chloroform, Tetrachlorkohlenstoff, Tetrachlorethylen; Alkohole, insbesondere aliphatische Alkohole wie Methanol, Ethanol, Propanol, Butanol; Ether und etherartige Verbindungen wie Dialkylether (Diethylether, Diisopropylether, tert.-Butyl-methylether usw.), Anisol, Dioxan, Tetrahydrofuran; Nitrile wie Acetonitril, Propionitril, N,N-dialkylierte Amide wie Dimethylformamid; Dimethylsulfoxid; Ketone wie Aceton, Diethylketon, Methylethylketon und Gemische solcher Lösungsmittel untereinander. In einigen Fällen kann auch einer der Reaktionspartner selbst als Lösungsmittel dienen.

Die Reaktionstemperaturen liegen normalerweise zwischen −5 und +200 °C, vorzugsweise zwischen 0 und 100 °C, wobei die Reaktionsdauer je nach gewählter Umsetzungstemperatur und Lösungsmittel zwischen wenigen Minuten und mehreren Tagen beträgt. In der Regel erfolgen die Reaktionen bei Normaldruck oder leichtem Überdruck. Die Gegenwart eines Reaktionskatalysators, z.B. Tetraalkylammoniumchlorid, Kaliumjodid und/oder Kronenäther kann von Vorteil sein.

Das Herstellungsverfahren ist ein Teil der Erfindung.

Die Ausgangsstoffe der Formeln II bis VII sind bekannt oder können nach an sich bekannten Methoden hergestellt werden.

Substituierte Säurehalogenide der Formel II sind z.B. durch Reaktion einer Verbindung der Formel VIII

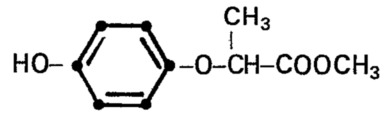

(VIII)

mit einem Trifluormethylpyridylderivat IX

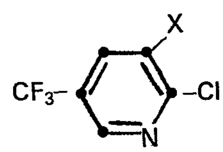

(IX)

zugänglich. Es entsteht zunächst unter HCl-Abspaltung ein Zwischenprodukt der Formel X

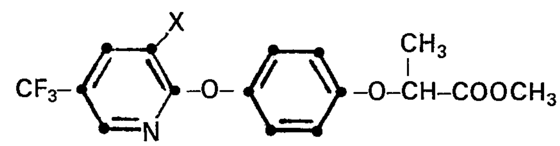

(X),

dessen Estergruppe auf übliche Art, z.B. in alkalischem Medium, zu einer Säuregruppe verseift und anschliessend beispielsweise mit einem geeigneten Halogenierungsmittel, wie $SOCl_2$, in ein Säurehalogenid der Formel II überführt werden kann. In den Formeln IX und X hat X die unter Formel I angegebenen Bedeutungen.

Verbindungen der Formel X zeigen herbizide Wirkung.

Verbindung VIII ist aus Hydrochinon und 2-Chlor-propionsäuremethylester in Gegenwart einer Base zugänglich.

Substituierte Trifluormethylpyridyloxy-parahydroxyphenylether der Formel IV können gemäss der Belgischen Patentschrift 862 325 und der DT-OS 2 847 662 oder 2 732 846 hergestellt werden.

Die Verbindungen der Formel I besitzen stets ein asymmetrisches Kohlenstoffatom C* in Nachbarstellung zur Methylgruppe des Propionsäurerestes.

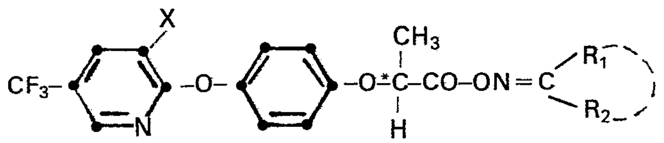

(I').

Bei der Synthese fallen die Wirkstoffe der Formel I normalerweise als racemisches Gemisch an und können auf übliche Art in ihre optischen Antipoden gespalten werden, so z.B. durch fraktionierte Kristallisation. Andererseits ist auch die Synthese einer optisch reinen Verbindung der Formel I möglich, wenn man bei deren Herstellung von einer optisch reinen 2-Halogenpropionsäure ausgeht. Die optischen Antipoden von I besitzen unterschiedliche biozide Wirkung, wobei die D-Form herbizid wirksamer ist.

Unabhängig von der genannten Isomerie können unsymmetrisch substituierte ($R_1 \neq R_2$) Wirkstoffe der Formel I in der syn- oder anti-Form vorliegen.

Sofern keine gezielte Synthese zur Isolierung reiner Isomeren durchgeführt wird, fällt ein Produkt der Formel I stets als Gemisch dieser möglichen isomeren Formen an.

Es ist bereits bekannt geworden, dass man bestimmte Oximester von Carbonsäuren als Herbizide verwenden kann (vgl. die offengelegten europäischen Patentanmeldungen Nr. 2 246 und 3 114 respektive die US-PS 4 200 587). Jedoch ist die herbizide Wirksamkeit besonders gegen schwer bekämpfbare Unkräuter nicht immer befriedigend.

Es wurde nun überraschenderweise gefunden, dass die trifluormethylierten neuen Pyridyloxyderivate der Formel I gemäss vorliegender Erfindung eine bessere Herbizidwirkung besitzen als die zitierten Verbindungen und bei pre- und postemergenter Anwendung als Unkrautmittel eingesetzt werden können.

Die erfindungsgemässen Wirkstoffe der Formel I und die sie als aktive Komponente enthaltenden herbiziden Mittel sind insbesondere brauchbar zur selektiven Bekämpfung von schwer bekämpfbaren Ungräsern in Kulturpflanzenbeständen, besonders in dikotylen Kulturpflanzen, wie Soja und sämtliche kultivierten Leguminosen, Baumwolle, Zuckerrüben, Sonnenblumen, Raps und weitere Brassica-Arten. Sie können ebenfalls in perenierenden Kulturen wie Reben, Obstanlagen (Äpfel, Birnen, Pfirsiche, Aprikosen), Citruskulturen sowie Plantagen von Kautschuk oder Ölpalmen zur Ungrasbekämpfung eingesetzt werden. Die erfindungsgemässen Verbindungen sind also gegenüber vielen Kulturpflanzen gut verträglich und gegen Ungräser sehr wirksam.

Darüber hinaus besitzen gewisse erfindungsgemässe Wirkstoffe in geeigneter Aufwandmenge auch günstige wachstumsregulierende Effekte (Wuchshemmung). Insbesondere hemmen sie auch das Wachstum von dicotylen Pflanzen. Beispiele für die nutzbringende Anwendung der erfindungsgemässen Verbindungen sind z.B. die Reduktion des vegetativen Wachstums bei Soja und ähnlichen Leguminosen, was zu einer Ertragssteigerung dieser Kultur führt, die Hemmung des unerwünschten Wachstums von Geiztrieben bei Tabak, dessen Haupttrieb man geschnitten hat, was der Ausbildung grösserer und schönerer Blätter zugute kommt, oder die Hemmung des Wachstums von dikotyledonen Pflanzen, wie Obstbäume, Zierbäume, Gebüsche und Hecken, zwecks Einsparung an Schnittarbeit.

Die neuen Verbindungen vorliegender Erfindung sind wenig giftig für Warmblüter und ihre Applikation wirft keine Probleme auf. Die Aufwandmenge liegt im allgemeinen zwischen 0,005 und 5 kg Aktivsubstanz pro Hektar, vorzugsweise zwischen 0,05 und 2 kg Aktivsubstanz je Hektar.

Die Wirkstoffe der Formel I als Bestandteile herbizider Mittel besitzen schon in niedrigen Aufwandmengen eine ausgesprochene herbizide Wirkung mit hoher Selektivität in Nutzpflanzenkulturen, vor allem dikotylen wie Baumwolle, Raps, Soja und Zuckerrüben.

Die Mittel mit Wirkstoffen der Formel I können pre-emergent auf angesäte Kulturflächen, oder aber vorzugsweise post-emergent in den genannten aufgelaufenen Kulturen als Kontaktherbizide appliziert werden.

So wird in Sojabohnenkulturen bei pre-emergenter Anwendung von Mitteln, die einen Wirkstoff der Formel I enthalten, hervorragende selektive Herbizidwirkung erzielt.

Die vorliegende Erfindung betrifft somit auch herbizide und/oder wachstumsregulierende Mittel, die als Aktivsubstanz eine Verbindung der Formel I enthalten, sowie die Herstellung dieser Mittel und die Verwendung der Mittel bzw. der Aktivstoffe zur selektiven Bekämpfung von Unkräutern in Kulturpflanzen und/oder zur Hemmung des vegetativen Pflanzenwachstums.

Der Gehalt an Wirkstoff im handelsfähigen Mittel liegt zwischen 0,1 bis 95 Gewichtsprozent, bevorzugt zwischen 1 bis 80 Gewichtsprozent. Anwendungskonzentrationen können, je nach Problemstellung, bis hinab auf 0,001 Gewichtsprozent verdünnt sein.

Die Herstellung erfindungsgemässer Mittel erfolgt in an sich bekannter Weise durch inniges Vermischen und Vermahlen von Wirkstoffen der allgemeinen Formel I mit geeigneten Trägerstoffen und/oder Verteilungsmitteln, gegebenenfalls unter Zusatz von gegenüber den Wirkstoffen inerten Antischaum-, Netz-, Dispersions- und/oder Lösungsmitteln. Die Wirkstoffe können in den folgenden Aufarbeitungsformen vorliegen und angewendet werden:

Feste Aufarbeitungsformen:

Stäubemittel, Streumittel, Granulate, Umhüllungsgranulate, Imprägnierungsgranulate und Homogengranulate;

In Wasser dispergierende Wirkstoffkonzentrate:

Spritzpulver (wettable powder), Pasten, Emulsionen; Emulsionskonzentrate;

Flüssige Aufarbeitungsformen:

Lösungen.

Bevorzugt werden Emulsionskonzentrate als Aufbereitungsform verwendet.

Den beschriebenen erfindungsgemässen Mitteln lassen sich andere biozide Wirkstoffe oder Mittel beimischen. So können die neuen Mittel ausser den genannten Verbindungen der allgemeinen Formel I z.B. Insektizide, Fungizide, Bakterizide, Fungistatika, Bakteriostatika, Nematozide, oder weitere Herbizide zur Verbreiterung des Wirkungsspektrums enthalten.

Die nachfolgenden Beispiele dienen zur näheren Erläuterung der Erfindung. Temperaturen sind in Celsiusgraden angegeben. Prozente und Teile beziehen sich auf Gewicht. Sofern nicht anders vermerkt, ist bei der Nennung eines Wirkstoffs der Formel I stets das racemische Gemisch gemeint.

Herstellungsbeispiele:

Beispiel 1:

Herstellung von

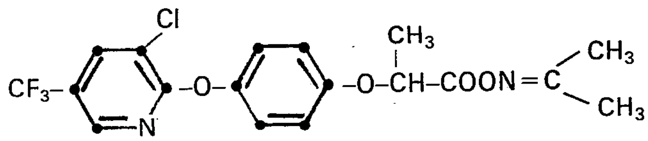

α-[-4-(3-Chlor-5-trifluormethyl-pyridyl-2-oxy)-phenoxy-]propionsäuremethylester

a) Herstellung des Zwischenproduktes:

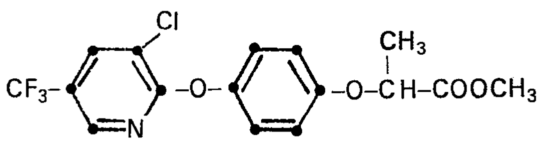

α-[4-(3-Chlor-5-trifluormethyl-pyridyl-2-oxy)- phenoxy-]propionsäuremethylester

53 g (0,27 Mol) α-(4-Hydroxy-phenoxy)-propionsäuremethylester und 47 g (0,34 Mol) Kaliumcarbonat werden in 300 ml Äthylmethylketon 1 Stunde bei +40°C gerührt, anschliessend werden 2 g Benzyltriethylammoniumchlorid und 58,3 g (0,27 Mol), 2,3-Dichlor-5-trifluormethlypyridin zugesetzt und das Reaktionsgemisch 10 Stunden unter Rückfluss erhitzt. Gebildetes Salz wird abfiltriert und das Filtrat eingedampft. Man erhält 85,5 g (85% der Theorie) α- [4-(3-Chlor-5-trifluormethyl-pyridyl-2-oxy-) phen--oxy-]propionsäuremethylester

b) Herstellung eines weiteren Zwischenproduktes:

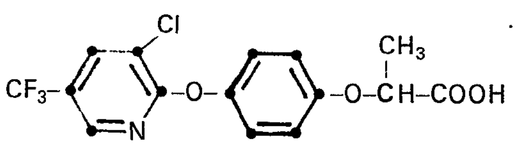

α-[4-(3-Chlor-5-trifluormethyl-pyridyl-2-oxy)- phenoxy]-propionsäure

37,5 g des nach a) hergestellten Esters werden in 120 ml 2 N Natronlauge 2 Stunden bei + 70 °C gerührt. Nach dem Abkühlen auf Raumtemperatur wird das Gemisch mit konzentrierter Salzsäure angesäuert und mit Diethylether extrahiert. Die vereinigten Extrakte werden über Magnesiumsulfat getrocknet, filtriert und das Lösungsmittel im Vakuum entfernt. Man erhält 33 g (91,3% d.Th.) α- [4- (3-Chlor-5-trifluormethyl-pyridyl-2-oxy)-phenoxy-]propionsäure mit einem Schmelzpunkt von 104–106°C.

c) Herstellung des Zwischenproduktes:

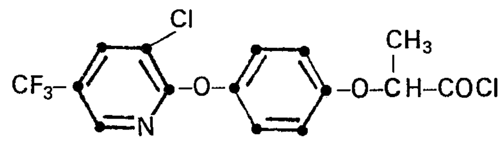

α-[4-(3-Chlor-5-trifluormethyl-pyridyl-2-oxy)- phenoxy]-propionsäurechlorid

18,1 g (0,05 Mol) der nach b) hergestellten Säure und 15 ml Thionylchlorid werden 5 Stunden bei + 60 °C gerührt. Überschüssiges Thionylchlorid wird im Vakuum entfernt, der Rückstand in Toluol aufgenommen und bis zur Trockne eingedampft. Man erhält 19,8 g α-[4-(3- Chlor- 5-trifluormethyl-pyridyl-2-oxy-)-phen- oxy-[-propionsäurechlorid als gelbes Öl.

d) Herstellung des Endproduktes:

11,8 g (0,03 Mol) des nach c) hergestellten Säurechlorids werden in 40 ml Toluol gelöst. Bei 0 °C wird eine Lösung von 2,4 g (0.034 Mol) Acetonoxim und 4 g (0,04 Mol) Triethylamin zugetropft. Man rührt das Gemisch 2 Stunden bei Raumtemperatur, filtriert das ausgefallene Salz ab und dampft das Filtrat ein. Der Rückstand wird in Ethylacetat/Hexan (1:1) aufgenommen und über Kieselgel filtriert. Man erhält 9,2 g (73% d.Th.) α-[4- (3- Chlor-5- trifluormethyl-pyridyl-2-oxy-)-phenoxy-] -propionsäure-acetonoximester mit einem Schmelzpunkt von 59–62 °C. (Verbindung No. 2)

Auf analoge Weise lassen sich auch die nachfolgenden Verbindungen der Formel I herstellen.

Tabelle 1: Verbindungen der Formel I

| Verb. Nr. | X | $R_1$ | $R_2$ | Physik. Konstante |
|---|---|---|---|---|
| 1 | Cl | H | $CH_3$ | |
| 2 | Cl | $CH_3$ | $CH_3$ | Smp. 59–62 °C |
| 3 | Br | $CH_3$ | $CH_3$ | |
| 4 | Cl | H | $C_2H_5$ | |
| 5 | CN | $CH_3$ | $CH_3$ | |
| 6 | Cl | $C_2H_5$ | $C_2H_5$ | $n_D^{25}$ 1.5250 |
| 7 | Cl | $CH_3$ | $(n)C_3H_7$ | |
| 8 | Cl | $CH_3$ | $C_2H_5$ | $n_D^{30}$ 1.5220 |
| 9 | Br | $(i)C_3H_7$ | $(i)C_3H_7$ | |
| 10 | CN | $CH_3$ | $(i)C_3H_7$ | |
| 11 | Cl | H | $C_6H_5$ | $n_D^{35}$ 1.5620 |
| 12 | Cl | H | $C_6H_4Cl$ (4) | |
| 13 | Cl | $(n)C_3H_7$ | $(n)C_3H_7$ | |
| 14 | Cl | $CH_3$ | $C_6H_5$ | |
| 15 | Cl | $CH_3$ | $C_6H_4Cl$ (3) | |
| 16 | Br | $CH_3$ | $C_6H_5$ | |
| 17 | Cl | $CH_3$ | $(n)C_4H_9$ | |
| 18 | Cl | $CH_3$ | $-CH=CH-CH_3$ | |
| 19 | Cl | $CH_3$ | $-CH=C(CH_3)_2$ | |
| 20 | H | H | tert. $C_4H_9$ | |
| 21 | H | $CH_3$ | $CH_3$ | $n_D^{30}$ 1.5165 |
| 22 | H | $CH_3$ | $C_2H_5$ | $n_D^{30}$ 1.5135 |
| 23 | H | $C_2H_5$ | $C_2H_5$ | |
| 24 | Cl | $-CH_2)_4-$ | | $n_D^{30}$ 1.5320 |
| 25 | Cl | $-(CH_2)_5-$ | | $n_D^{25}$ 1.5327 |
| 26 | Cl | $-(CH_2)_6$ | | |
| 27 | H | $-(CH_2)_4-$ | | |
| 28 | F | $CH_3$ | $CH_3$ | |
| 29 | F | $CH_3$ | $C_2H_5$ | |
| 30 | F | $C_2H_5$ | $C_2H_5$ | |
| 31 | Cl | $CH_3$ | $C(CH_3)_3$ | |
| 32 | Cl | $CH_3$ | $CH_2CH(CH_3)_2$ | |

Formulierungsbeispiele:

Beispiel 2: Emulsionskonzentrate

Zur Herstellung eines a) 10%igen und b) 25%igen und c) 50%igen Emulsionskonzentrates werden die folgenden Stoffe verwendet:

| | a) | b) | c) |
|---|---|---|---|
| Wirkstoff der Formel I | 10% | 25% | 50% |
| Ca-Dodecylbenzolsulfonat | 5% | 3% | 4% |
| Ricinusöl-polyäthylenglykol-äther (36 Mol AeO) | 5% | 7% | – |
| Tributylphenoxyl-polyäthylen-glykoläther (30 Mol AeO) | – | – | 6% |
| Mineralöl mit hohem Paraffin-gehalt | 10% | – | – |
| Cyclohexanon | – | – | 15% |
| Xylolgemisch | 70% | 65% | 25% |

Aus solchen Konzentraten können durch Verdünnen mit Wasser Emulsionen jeder gewünschten Konzentration hergestellt werden.

Die neuen 4-Pyridyloxy-phenoxy-propionsäuren und ihre Derivate der Formel I, sowie die sie enthaltenden Mittel besitzen eine ausgezeichnete selektiv-herbizide Wirkung gegen Unkräuter in den verschiedensten Kulturpflanzenbeständen. Einige davon haben ebenfalls eine das Pflanzenwachstum regulierende Wirkung.

Obwohl die neuen Wirkstoffe der Formel I bei pre- und post-emergenter Anwendung wirksam sind, wird bei der pre-emergenten Applikation im Gegensatz zu vielen bekannten Phenoxyphenoxy-Herbiziden eine vorzügliche Wirkung erzielt.

Bevorzugt werden die neuen Wirkstoffe als z.B. 50%ige oder 25%ige emulgierbare Konzentrate formuliert und mit Wasser verdünnt, auf die

Pflanzenbestände post-emergent oder direkt auf den Boden pre-emergent bzw. inkorporiert angewendet.

Zum Nachweis der Brauchbarkeit als Herbizide (pre-emergent) und als Wuchshemmer dienten die folgenden Testmethoden:

Beispiel 3:

Pre-emergente Herbizid-Wirkung

Im Gewächshaus wird unmittelbar nach der Einsaat der Versuchspflanzen in Töpfe von 11 cm Durchmesser die Erdoberfläche mit einer wässrigen Suspension der Wirkstoffe, erhalten aus einem 25%igen Emulsionskonzentrat behandelt. Es wurden zwei verschiedene Konzentrationsreihen angewendet, entsprechend 1 und 0,5 kg Wirksubstanz pro Hektar. Die Töpfe werden im Gewächshaus bei 22–25 °C und 50–70% relativer Luftfeuchtigkeit gehalten und der Versuch nach 3 Wochen ausgewertet und die Resultate nach folgender Notenskala bonitiert (9-er Index):

1 = Pflanzen nicht gekeimt oder total abgestorben

2–8 = Zwischenstufen der Schädigung

9 = Pflanzen ungeschädigt (wie unbehandelte Kontrolle).

Die Versuchsergebnisse sind untenstehend zusammengefasst:

| Verbindung Nr. | 2 | | 8 | | 21 | | 22 | |
|---|---|---|---|---|---|---|---|---|
| Aufwandmengen kg/ha | 1 | 0.5 | 1 | 0.5 | 1 | 0.5 | 1 | 0.5 |
| Pflanze | | | | | | | | |
| avena fatua | 2 | 2 | 1 | 2 | 7 | 0 | 3 | 9 |
| bromus tectorum | 1 | 1 | 1 | 2 | 2 | 2 | 1 | 6 |
| lolium perenne | 1 | 1 | 1 | 1 | 1 | 2 | 1 | 1 |
| alopecurus myosuroides | 2 | 2 | 1 | 1 | 1 | 1 | 1 | 2 |
| digitaria sanguinalis | 1 | 1 | 1 | 1 | 1 | 2 | 1 | 2 |
| echinochloa crus galli | 1 | 1 | 1 | 1 | 1 | 1 | 1 | 1 |
| sorghum halepense | 1 | 1 | 1 | 1 | 1 | 1 | 1 | 1 |
| rottboellia exaltata | 1 | 1 | 1 | 1 | 1 | 2 | 1 | 1 |
| Soja | 8 | 8 | 9 | 9 | 9 | 9 | 9 | 9 |
| Zuckerrübe | 9 | 9 | 9 | 9 | 9 | 9 | 9 | 9 |
| Baumwolle | 9 | 9 | 9 | 9 | 9 | 9 | 9 | 9 |

Beispiel 4:

Herbizide Wirkung bei Applikation der Wirkstoffe nach dem Auflaufen der Pflanzen (post-emergent).

Verschiedene Kulturpflanzen und Unkräuter werden aus den Samen in Töpfen im Gewächshaus aufgezogen, bis sie das 4- bis 6-Blatt-Stadium erreicht haben. Dann werden die Pflanzen mit einer wässrigen Wirkstoffemulsion gespritzt, die 0,25 kg pro Hektar entspricht. Die behandelten Pflanzen werden dann bei optimalen Bedingungen von Licht, Begiessung, Temperatur (22–25 °C) und Luftfeuchtigkeit (50–70% relativ) gehalten. 15 Tage nach Behandlung erfolgte die Auswertung der Versuche nach oben gegebenem Index. Die Ergebnisse sind untenstehend zusammengefasst:

| Verbindung Nr. | 2 | 8 | 21 | 22 |
|---|---|---|---|---|
| Pflanze | | | | |
| avena fatua | 1 | 1 | 6 | 7 |
| bromis tectorum | 2 | 2 | 3 | 4 |
| lolium perenne | 1 | 1 | 3 | 3 |
| alopecurus myosuroides | 1 | 1 | 4 | 2 |
| digitaria sanguinalis | 1 | 1 | 2 | 1 |
| echinochloa crus galli | 1 | 1 | 1 | 1 |
| sorghum halepense | 1 | 1 | 1 | 1 |
| rottboellia exaltata | 1 | 1 | 5 | 2 |
| Soja | 9 | 9 | 9 | 9 |
| Zuckerrübe | 7 | 8 | 9 | 9 |
| Baumwolle | 9 | 9 | 9 | 9 |

Beispiel 5:

Ertragssteigerung bei Sojabohnen

In Kunststoffbehältern mit Erde-Torf-Sand-Gemisch (6:3:1) wurden Sojabohnen der Sorte «Hark» in der Klimakammer angesät. Durch optimale Temperaturwahl, Beleuchtung, Düngergabe und Bewässerung konnten sich nach ca. 5 Wochen die Pflanzen zu dem 5–6 Trifoliate-Blattstadium entwickeln. Zu diesem Zeitpunkt wurden die Pflanzen mit der wässrigen Spritzbrühe eines Wirkstoffes bis zur guten Benetzung besprüht. Die Wirkstoffkonzentration betrug 10, 50, 100 und 500 ppm Aktivsubstanz. Die Auswertung erfolgte ca. 5 Wochen nach der Applikation.

Die Verbindung No. 2 erzielte bei Wirkstoffkonzentrationen von 50 und 500 ppm gegenüber unbehandelten Kontrollpflanzen eine geringe Reduktion des Längenwachstums, während der Schotenansatz etwa um 4–7% gesteigert war.

**Patentansprüche**

1. Verbindungen der Formel I

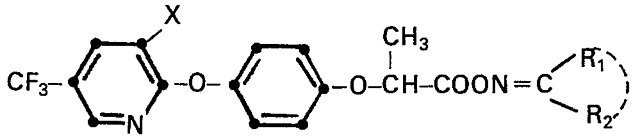 (I),

worin X für Wasserstoff, Halogen oder Pseudohalogen steht; $R_1$ Wasserstoff oder eine unsubstituierte oder durch Halogen, Hydroxy oder $C_1$–$C_3$-Alkoxy substituierte $C_1$–$C_6$-Alkyl- oder Phenylgruppe bedeutet; $R_2$ eine unsubstituierte oder eine durch Halogen, Hydroxy oder $C_1$–$C_3$-Alkoxy substituierte $C_1$–$C_6$-Alkyl-, $C_2$–$C_6$-Alkenyl-, $C_2$–$C_6$-Alkinyl- oder Phenylgruppe bedeutet; oder $R_1$ und $R_2$ zusammen für eine unsubstituierte oder eine durch $C_1$–$C_3$-Alkyl substituierte Tetramethylen-, Pentamethylen- oder Hexamethylenbrücke stehen.

2. Verbindungen der Formel I nach Anspruch 1, dadurch gekennzeichnet, dass X für Chlor oder Brom steht und $R_1$ sowie $R_2$ die unter Formel I angegebenen Bedeutungen haben.

3. Verbindungen der Formel I nach Anspruch 1, dadurch gekennzeichnet, dass X für Wasserstoff steht und $R_1$ sowie $R_2$ die unter Formel I angegebenen Bedeutungen haben.

4. Verbindungen der Formel I nach Anspruch 1, dadurch gekennzeichnet, dass X für Chlor steht, $R_1$ und $R_2$ unabhängig voneinander $C_1$–$C_3$-Alkyl bedeuten.

5. α-[4-(3-Chlor-5-trifluormethyl-pyridyl-2-oxy)-phenoxy]-propionsäureacetoximester, nach Anspruch 1.

6. α-[4-(3-Chlor-5-trifluormethyl-pyridyl-2-oxy)-phenoxy]-propionsäure-methylethylketonoximester, nach Anspruch 1.

7. α-[4-(3-Chlor-5-trifluormethyl- pyridyl-2-oxy)-phenoxy]-propionsäurediethylketonoximester, nach Anspruch 1.

8. Verfahren zur Herstellung einer Verbindung der Formel I, Anspruch 1, dadurch gekennzeichnet, dass man in einem inerten organischen Lösungsmittel, gegebenenfalls in Gegenwart eines säurebindenden Mittels ein substituiertes Säurehalogenid der Formel II

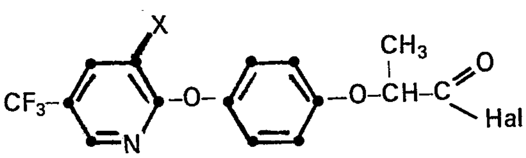 (II)

worin X die in Anspruch 1 gegebene Bedeutung hat und Hal für ein Halogenatom steht, mit einem Oxim der Formel III

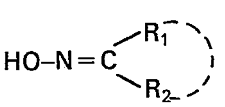 (III)

worin $R_1$ und $R_2$ die im Anspruch 1 gegebenen Bedeutungen haben, reagieren lässt.

9. Verfahren zur Herstellung einer Verbindung der Formel I, Anspruch 1, dadurch gekennzeichnet, dass man in einem inerten organischen Lösungsmittel, gegebenenfalls in Gegenwart eines säurebindenden Mittels eine Verbindung der Formel IV

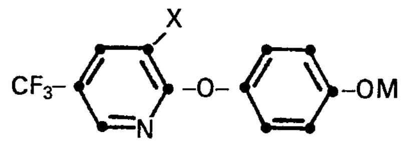 (IV)

worin X die im Anspruch 1 gegebene Bedeutung hat und M für Wasserstoff oder ein Alkalimetall- oder Erdalkalimetall-Kation steht, mit einem α-Halogenpropionsäureoximester der Formel V

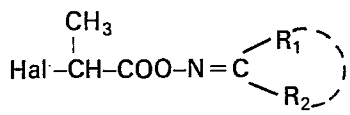 (V)

umsetzt, worin $R_1$ und $R_2$ die im Anspruch 1 gegebenen Bedeutungen haben und Hal für ein Halogenatom steht.

10. Verfahren zur Herstellung einer Verbindung der Formel I, Anspruch 1, dadurch gekennzeichnet, dass man in einem inerten organischen Lösungsmittel, gegebenenfalls in Gegenwart eines säurebindenden Mittels eine Verbindung der Formel VI

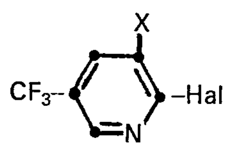 (VI)

worin X die im Anspruch 1 gegebene Bedeutung hat und Hal für ein Halogenatom steht, mit einem Milchsäureoximester der Formel VII

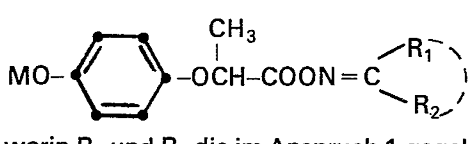 (VII)

worin $R_1$ und $R_2$ die im Anspruch 1 gegebene Bedeutung haben und M für Wasserstoff oder ein Alkalimetall- oder Erdalkalimetallkation steht, umsetzt.

11. Verfahren nach einem der Ansprüche 8 bis 10, dadurch gekennzeichnet, dass in den Formeln II, V und VI Hal für Chlor oder Brom steht und M in den Formeln IV und VII ein Natrium- oder Kaliumkation bedeutet.

12. Verfahren nach einem der Ansprüche 8 bis 10, dadurch gekennzeichnet, dass die Reaktion bei Temperaturen von −5 °C bis +200 °C durchgeführt wird.

13. Herbizide und/oder pflanzenwuchsregulierendes Mittel, dadurch gekennzeichnet, dass es als aktive Komponente ein Pyridyloxy-phenoxypropionsäurederivat der Formel I nach Anspruch 1 enthält.

14. Verwendung der Pyridyloxy-phenoxy-alkancarbonsäurederivate der Formel I nach Anspruch 1, oder sie enthaltender Mittel zur selektiven Bekämpfung von Unkräutern in Kulturpflanzungen.

15. Die Verwendung der Pyridyloxy-phenoxy-propionsäurederivate der Formel I nach Anspruch 1 oder sie enthaltender Mittel zur selektiven Bekämpfung von Unkräutern in Kulturen von Sojabohnen und Zuckerrüben.

16. Die Verwendung der Pyridyloxy-phenoxypropionsäurederivate der Formel I, Anspruch 1, oder sie enthaltenden Mittel zur Ertragssteigerung in Kulturen von Sojabohnen.

17. Verfahren zur selektiven Bekämpfung von Unkräutern in Kulturpflanzen, dadurch gekennzeichnet, dass man 0,005 bis 5 kg einer Aktivsubstanz der Formel I nach Anspruch 1 pro Hektar der Anbaufläche appliziert.

**Revendications**

1. Composés de formule I

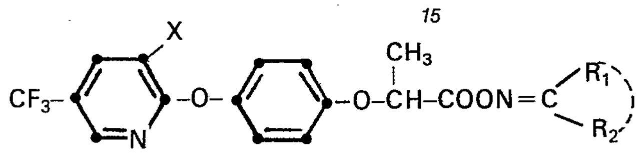

(I),

ou X représente un hydrogène, un halogène ou un pseudohalogène; $R_1$ représente un hydrogène ou un groupe alcoyle en $C_1$ à $C_6$ ou phényle non substitué ou substitué par un halogène, un hydroxy ou un alcoxy en $C_1$ à $C_3$; $R_2$ représente un groupe alcoyle en $C_1$ à $C_6$, alcényle en $C_2$ à $C_6$, alcynyle en $C_2$ à $C_6$ ou phényle non substitué ou substitué par un halogène, un hydroxy ou un alcoxy en $C_1$ à $C_3$; ou $R_1$ et $R_2$ représentent ensemble un pont tétraméthylène, pentaméthylène ou hexamethylène substitué par un alcoyle en $C_1$ à $C_3$.

2. Composés de formule I selon la revendication 1, caractérisé en ce que X représente un chlore ou un brome et $R_1$ ainsi que $R_2$ ont les significations données pour la formule I.

3. Composés de formule I selon la revendication 1, caractérisé en ce que X représente un hydrogène et $R_1$ ainsi que $R_2$ ont les significations données pour la formule I.

4. Composés de formule I selon la revendication 1, caractérisés en ce que X représente un chlore, $R_1$ et $R_2$ représentent indépendamment l'un de l'autre un alcoyle en $C_1$ à $C_3$.

5. Acétoximester de l'acide α-[4-(3-chloro-5-trifluorméthyl-pyridyl-2-oxy)-phénoxy]-propionique selon la revendication 1.

6. Méthyléthylcètonoximester de l'acide α-[4-(3 - chloro - 5 - trifluorométhyl - pyridyl - 2-oxy)-phénoxy]-propionique selon la revendication 1.

7. Diéthylcétonoximester de l'acide α-[4-(3-chloro-5-trifluorométhyl-pyridyl-2-oxy)-phénoxy]-propionique selon la revendication 1.

8. Procédé de préparation d'un composé de formule I de la revendication 1, caractérisé en ce qu'on fait réagir dans un solvant organique inerte, éventuellement en présence d'un agent liant acide, un halogénure d'acide substitué de formule II

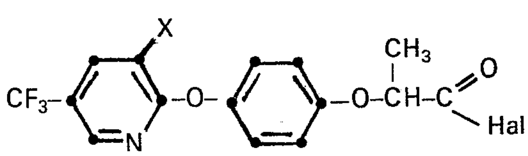

(II)

où X a la signification donnée dans la revendication 1 et Hal représente un atome d'halogène, avec un oxime de formule III

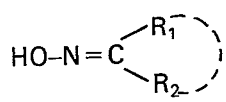

(III)

où $R_1$ et $R_2$ ont les significations données dans la revendication 1.

9. Procédé de préparation d'un composé de formule I de la revendication 1, caractérisé en ce qu'on fait réagir dans un solvant organique inerte, éventuellement en présence d'un agent liant acide, un composé de formule IV

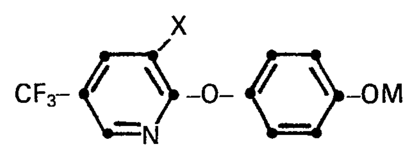

(IV)

où X a la signification donnée dans la revendication 1 et M représente un hydrogène ou un cation de métal alcalin ou de métal alcalino-terreux, avec un oximester d'acide α-halogènepropionique de formule V

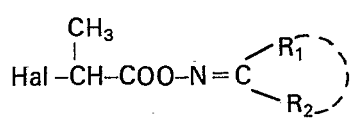

(V)

où $R_1$ et $R_2$ ont les significations données dans la revendication 1 et Hal représente un atome d'halogène.

10. Procédé de préparation d'un composé de formule I de la revendication 1, caractérisé en ce qu'on fait réagir dans un solvant organique inerte, éventuellement en présence d'un agent liant acide, un composé de formule VI

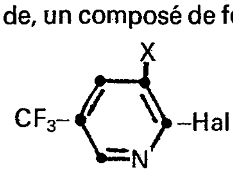

(VI)

où X a la signification donnée dans la revendication 1 et Hal représente un atome d'halogène, avec un oximester d'acide lactique de formule VII

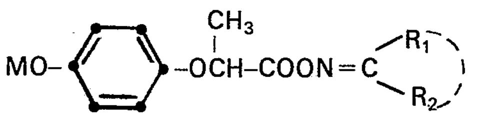
(VII)

où $R_1$ et $R_2$ ont la signification donnée dans la revendication 1 et M représente un hydrogène ou un cation de métal alcalin ou alcalino-terreux.

11. Procédé selon l'une des revendications 8 à 10, caractérisé en ce que dans les formules II, V et VI Hal représente un chlore ou un brome et M dans les formules IV et VII représente un cation sodium ou potassium.

12. Procédé selon l'une des revendications 8 à 10, caractérisé en ce que la réaction est conduite à des températures allant de −5 °C à +200 °C.

13. Agent herbicide et/ou régulateur de croissance des plantes, caractérisé en ce qu'il contient comme composant actif un dérivé d'acide pyridyloxy-phénoxy-propionique de formule I selon la revendication 1.

14. Application des dérivés d'acide pyridyloxyphénoxy-alcanecarboxyliques de formule I selon la revendication 1 ou des agents qui les contiennent à la lutte sélective contre les mauvaises herbes dans les plantes cultivées.

15. Application des dérivés d'acide pyridyloxyphénoxy-propionique de formule I selon la revendication 1 ou des agents qui les contiennent à la lutte sélective contre les mauvaises herbes dans les cultures de soja et de betterave sucrière.

16. Application des dérivés d'acide pyridyloxyphénoxy-propionique de formule I de la revendication 1 ou des agents qui les contiennent à l'accroissement du rendement dans les cultures de soja.

17. Procédé de lutte sélective contre les mauvaises herbes dans les cultures, caractérisé en ce qu'on applique de 0,005 à 5 kg d'une matière active de formule I selon la revendication 1 par ha de surface cultivée.

## Claims

1. A compound of the formula I

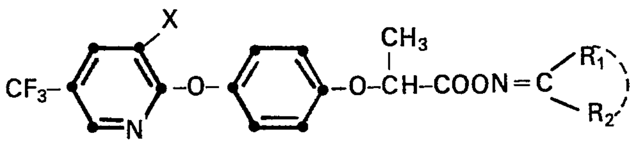
(I),

wherein

X is hydrogen, halogen or pseudo halogen,

$R_1$ is hydrogen or a $C_1$–$C_6$-alkyl or phenyl group each of which is unsubstituted or substituted by halogen, hydroxyl or $C_1$–$C_3$-alkoxy,

$R_2$ is a $C_1$–$C_6$-alkyl, $C_2$–$C_6$-alkenyl, $C_2$–$C_6$-alkynyl or phenyl group, each of which is unsubstituted or substituted by halogen, hydroxyl or $C_1$–$C_3$-alkoxy, or

$R_1$ and $R_2$ together form a tetramethylene, pentamethylene or hexamethylene bridge, each of which is unsubstituted or substituted by $C_1$–$C_3$-alkyl.

2. A compound of the formula I according to Claim 1, wherein X is chlorine or bromine, and $R_1$ and $R_2$ have the meanings defined under the formula I.

3. A compound of the formula I according to Claim 1, wherein X is hydrogen, an $R_1$ and $R_2$ have the meanings defined under the formula I.

4. A compound of the formula I according to Claim 1, wherein X is chlorine, and $R_1$ and $R_2$ independently of one another are each $C_1$–$C_3$-alkyl.

5. α-[4-(3-Chloro-5-trifluoromethyl-pyridyl-2-oxy)-phenoxy]-propionic acid acetoxime ester, according to Claim 1.

6. α-[4-(3-Chloro-5-trifluoromethyl-pyridyl-2-oxy)-phenoxy]-propionic acid methyl ethyl ketoxime ester, according to Claim 1.

7. α -[4-(3-Chloro-5-trifluoromethyl-pyridyl-2-oxy)-phenoxy]-propionic acid diethyl ketoxime ester, according to Claim 1.

8. A process for producing a compound of the formula I, Claim 1, which process comprises reacting in an inert organic solvent, optionally in the presence of an acid-binding agent, a substituted acid halide of the formula II

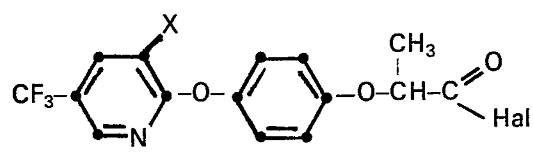
(II)

wherein X has the meaning defined in Claim 1 and Hal is a halogen atom, with an oxime of the formula III

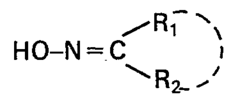
(III)

wherein $R_1$ and $R_2$ have the meaning defined in Claim 1.

9. A process for producing a compound of the formula I, Claim 1, which process comprises reacting in an inert organic solvent, optionally in the presence of an acid-binding agent, a compound of the formula IV

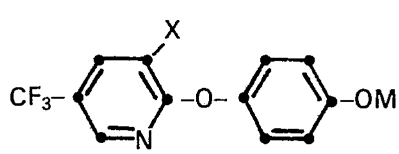
(IV)

wherein X has the meaning defined in Claim 1, and M is hydrogen or an alkali metal cation or alkaline-earth metal cation, with an α-halopropionic acid oxime ester of the formula V

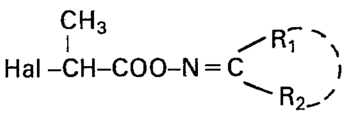
(V)

wherein $R_1$ and $R_2$ have the meanings defined in Claim 1 and Hal is a halogen atom.

10. A process for producing a compound of the formula I, Claim 1, which process comprises reacting in an inert organic solvent, optionally in the presence of an acid-binding agent, a compound of the formula VI

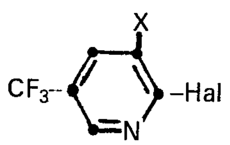
(VI)

wherein X has the meaning given in Claim 1, and Hal is a halogen atom, with a lactic acid oxime ester of the formula VII

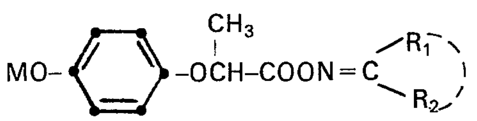
(VII)

wherein $R_1$ and $R_2$ have the meanings defined in Claim 1, and M is hydrogen or an alkali metal cation or alkaline-earth metal cation.

11. A process as claimed in any one of Claims 8 to 10, wherein, in the formulae II, V and VI, Hal is chlorine or bromine, and M in the formulae IV and VII is a sodium or potassium cation.

12. A process according to claims 8 to 10, wherein the reaction is performed at temperatures of −5 °C to +200 °C.

13. A herbicidal and/or plant-growth-regulating composition which contains, as active ingredient, a pyridyl-phenoxypropionic acid derivative of the formula I according to Claim 1.

14. A method of selectively combating weeds in crops of cultivated plants, which method comprises applying thereto or to the locus thereof a herbicidally effective amount of a pyridyloxy-phenoxy-alkanecarboxylic acid derivative of the formula I according to Claim 1, or of a composition containing it.

15. A method of selectively combating weeds in cultivated crops of soya-bean and sugar beet, which method comprises applying thereto or to the locus thereof a herbicidally effective amount of a pyridyloxy-phenoxy-propionic acid derivative of the formula I, Claim 1, or of a composition containing it.

16. A method of increasing the yields in cultivated crops of soya-bean, which method comprises applying thereto or to the locus thereof an effective amount of a pyridyloxy-phenoxy-propionic acid derivative of the formula I, Claim 1, or of a composition containing it.

17. A method of selectively combating weeds in crops of cultivated plants, which method comprises applying 0,005 to 5 kg of an active substance of the formula I, Claim 1, per hectare of the cultivated area.